Europäisches Patentamt

European Patent Office

Office européen des brevets

(11) Publication number: **0 164 397**
**B1**

# EUROPEAN PATENT SPECIFICATION

(45) Date of publication of patent specification: 04.07.90

(51) Int. Cl.⁵: **A 61 K 37/00, A 61 K 37/26**

(21) Application number: 85900286.7

(22) Date of filing: 13.11.84

(86) International application number:
PCT/US84/01859

(87) International publication number:
WO 85/02118 23.05.85 Gazette 85/12

(54) **A BUFFERED POLYOL-HORMONE MIXTURE FOR USE IN CHRONIC PARENTERAL HORMONE ADMINISTRATION.**

(30) Priority: 21.11.83 US 553572

(43) Date of publication of application:
18.12.85 Bulletin 85/51

(45) Publication of the grant of the patent:
04.07.90 Bulletin 90/27

(84) Designated Contracting States:
DE FR GB

(56) References cited:
DIABETOLOGIA, vol. 19, 1980, pages 1-9; W.D.
LOUGHEED et al.: " Insulin aggregation in
artificial delivery systems"

ARTIFICIAL SYSTEMS FOR INSULIN DELIVERY,
1983, pages 83-84, Raven Press., New York, US;
J. BRANGE et al.: "Properties of insulin in
solution"

The file contains technical information
submitted after the application was filed and
not included in this specification

(73) Proprietor: REGENTS OF THE UNIVERSITY OF
MINNESOTA
Morrill Hall 100 Church Street Southeast
Minneapolis Minnesota 55455 (US)

(72) Inventor: WIGNESS, Bruce, D.
2447 - 15th Avenue South
Minneapolis, MN 55404 (US)

(74) Representative: Hildyard, Edward Martin et al
Frank B. Dehn & Co. European Patent Attorneys
Imperial House 15-19 Kingsway
London WC2B 6UZ (GB)

Courier Press, Leamington Spa, England.

## Description

This invention relates to a method of maintaining and extending the bioactivity, while preventing precipitation, of protein hormone preparations, within drug delivery systems that depend on the fluidity of the infusate for proper function. One system of this type is the implantable infusion pump illustrated and described in Blackshear et al U.S. Patent No. i.e. US—A—3,731,681, the disclosure of which is incorporated herein by reference. In U.S. Patent No., i.e. US—A—4,439,181, issued March 27, 1984, of common ownership herewith, it is disclosed that the addition of a polyol, such as glycerol, increases the solubility of the protein (i.e., a hormone, such as insulin) in water without affecting its biological activity. The polyol solubilizes both native or denatured proteins and inhibits the action of precipitation agents, such as adverse temperatures. The disclosure of U.S. Patent No., i.e. US—A—4,439,181 is incorporated herein by reference.

The control of many cellular functions in an organism is via hormones, such as insulin, human growth hormone or glucagon, secreted in very small amounts by specific glands into the blood stream. These hormones have high affinity for specific sites on or in the membranes of the specific cells. The hormones are typically small proteins with a marginal solubility in blood. The hormone is functionally active in the hydrophobic lipid membrane. A typical hormone deficiency disease is diabetes where the beta cells in the pancreas have been destroyed or are relatively ineffective, and the medical treatment is the administration of insulin.

Recent studies have suggested that management of blood glucose in diabetics can be improved by administering insulin by pump on a continuous basis, or in pulses several minutes apart, rather than by intermittent injections as is now common practice. One factor limiting the length of time this kind of administration can be maintained has been precipitation of insulin in the flow passages of these pumps causing flow stoppage. The solution of this major probem has been the subject of intensive research by several different organizations and groups.

A further requirement of drug diluting fluids not necessarily related to nonprecipitability is stable bioavailability. A practical insulin diluting fluid suitable for an implantable drug pump must demonstrate no appreciable loss in bioactivity after one or more months of storage at body temperature in the pump drug reservoir.

Data accumulated since the filing of the application from which U.S. Patent No. i.e. US—A—4,439,181 issued has further confirmed the effectiveness of the addition of a polyol, such as gycerol, in inhibiting the precipitation of hormones, such as insulin. However, there is evidence of partial loss of activity of the hormone over the extended period of time it remains in the drug delivery device. The lowering of biological activity is believed to be a time- and temperature-dependent propensity of the hormone to form soluble, higher molecular weight hormone polymers or oligomers.

An alternative cause of loss of biological activity may be due to molecular alteration of the hormone due to pH conditions. For instance, an insulin diluting fluid that has a sufficiently alkaline pH may cause the cleavage of cystine sulfhydral linkages resulting in non-reversible loss of native tertiary structure. Although insulin does not generally precipitate as a consequence of such pH conditions (in fact solubility is enhanced), the unfolding of the molecule and subsequent random intermolecular and intramolecular reformation of the cystiene-cystiene bonds, results in a mixture of species with overall loss of bioactivity.

It has now been discovered that formation of polymers may be prevented and biological activity over an extended time period may be maintained by the addition of phosphate buffer at neutral pH, along with sodium bicarbonate.

Albisser and colleagues (Albisser, A. M., et al: Diabetes 29:241, 1980) describe the use in their portable peristaltic pump of the addition of 1.5% autologous serum to prevent the precipitation and loss of insulin potency that occurred without serum. However, use of 1.5% serum in animal studies at University of Minnesota Hospitals failed to prevent clogging of the capillary tubes of the pump of Patent No. 3,731,681 with insulin, possibly because of major differences in inner diameter between the capillary tubing of that pump and Albisser's delivery cannula.

Dorman et al U.S. Patent No. i.e. US—A—4,306,553 discloses that a surfactant, such as sodium lauryl sulfate, will prevent the precipitation of insulin in the pump's delivery cannula. However, this compound has not yet been proven safe when used intravenously in man. Its effect on long term maintenance of biological activity has not been determined.

Lougheed et al (Insulin Aggregation in Artificial Delivery Systems, Diabetologia 19:1—9, 1980) demonstrated that although sodium bicarbonate in concentrations of at least 23 mMolar is a solubilizing agent for insulin crystals, including it in regular insulin solutions (either neutral insulin or its thiol derivative) does not prevent precipitates from forming in *in vitro* tests designed to mimic drug pump conditions. There is no disclosure of the effect of sodium bicarbonate on insulin bioactivity.

Diabetalogica, *19* (1980), P1—9 and Artificial Systems for Insulin Delivery, *6* (1983) p83—88 also discuss insulin solutions and their delivery.

Broadly stated, this invention is directed to a method of maintaining the fluidity of protein hormone solutions for parenteral administration at a low flow rate to a chronically ill patient suffering from a protein-deficiency disease from an implanted pressure actuated drug delivery device without loss of biological activity during the extended time period the solution is stored in the drug delivery device. A polyol, such as glycerol, is mixed with protein hormone solution prior to the injection of the solution into the drug storage chamber of the delivery device, for thorough dissolution and uniform distribution through the solution, along with a buffer to extend and maintain bioactivity of the drug. The polyol is added in an amount sufficient to prevent precipitation of the protein hormone during long-term storage in the drug delivery device of up to several weeks. The buffer is added in approximately physiologic concentrations. Thereafter, the protein hormone-polyol-buffer solution is injected into the drug storage chamber by injection through the patient's skin. As the solution is discharged from the delivery device by the constant pressure exerted upon the storage chamber, its low rate of flow is controlled by a restricted fluid passage. The solution is conveyed to an infusion site by tubular passage means, such as a small diameter catheter.

Detailed Description of the Preferred Embodiment

According to the invention, a polyol solution, such as a solution of glycerol, is admixed with a solution of protein hormone, such as a standard injectable hormone solution, so that the final concentration of polyol in the solution is from 10 to 90% by volume, and preferably about 40 to 80%, sufficient to prevent precipitation of the protein hormone after charging into the drug storage chamber of a pressure actuated drug delivery device, such as the infusion pump of U.S. Patent No. i.e. US—A—3,731,681.

At the same time, to extend and maintain the biological activity of the protein hormone during its period of storage in the drug delivery device, a small amount of a physiologically compatible buffer system is added to the solution in concentration between 1 mMolar and 1000 mMolar sufficient to maintain its pH within 1 unit of optimum pH for the particular protein hormone. The optimum pH for sulfated insulin is 3.5; that for standard neutral insulin is 7.4. The objective is to avoid the isoelectric point of 5.4, which causes precipitation, and alkaline pH in the range of 8.0 and above, which is denaturing. The addition of a buffer capable of maintaining the pH in the desired range was found to reduce both polymerization and loss of bioactivity. Whether this stabilizing effect is related to avoiding mechanisms associated with pH shifts toward the isoelectric point or alkaline pH shifts is uncertain. However, samples with pH shifts in both directions have been prepared and polymerization has been detected in each case.

Suitable buffers include the sodium phosphate, sodium bicarbonate, sodium acetate, sodium citrate, and other similar physiologically compatible buffer systems. In anticipation of eventual testing for clearance by the FDA for clinical intravenous use, the sodium phosphate buffer system at a pH of 7.4 (physiologic intravascular pH) in a concentration currently approved (54 mMolar, physiologic intracellular concentration), in combination with 12 mM sodium bicarbonate, was selected for testing.

The protein hormone polyol-buffer solution is injected into the drug storage chamber of the drug delivery device of a patient suffering from a protein hormone-deficiency disease. From the storage chamber the solution is discharged through a fluid restrictor at a low flow rate of from 0.1 cc to 15 cc per day, depending upon the needs of the patient, and passed into the blood stream at the desired infusion site. Upon passage into the blood stream, the solution is immediately diluted to below 0.15% by weight to rapidly return the protein hormone to its normal state where it may function normally with no loss of activity.

The invention is illustrated by the following examples:

Example 1

Infusion of an insulin/glycerol/bicarbonate mixture in diabetic human subjects. In nine patients with Type II diabetes, infusion pumps (Infusaid™ Model 100, Infusaid Corporation, Norwood, MA) were implanted subcutaneously in the anterior chest wall, and the delivery cannulas were threaded into the superior vena cava via the subclavian vein in all cases. Two solutions were prepared. One contained 20 or 40 units per ml Neutral Regular porcine insulin (Lilly), 0.2 per cent (w/v) phenol, 12 mM sodium bicarbonate and 80 per cent (v/v) glycerol. The other contained no insulin but also contained 0.2 per cent (w/v) phenol, 12 mM sodium bicarbonate and 80 per cent (v/v) glycerol. The two solutions were of identical viscosity. Immediately before use, the two solutions were combined in appropriate amounts to provide the appropriate final infusate insulin concentration. This procedure was found to be necessary because of the major alterations in pump flow rate that occur with minimal alterations in the viscosity of the 80 per cent glycerol solutions.

No instances of pump occlusion or significant flow rate reductions have occurred after more than 460 patient-weeks of insulin/glycerol infusion in nine subjects in whom Model 100 pumps were implanted. Pump occlusion occurred in our first patient in whom a lower glycerol concentration was used in a Model 400 pump, which contains an auxiliary side port. The insulin delivered was clearly biologically active, as evidenced by the excellent glycemic control achieved. However, refill intervals of approximately 10 to 14 days were necessary due to a noticeable loss of bioactivity which was accomplished by changes of infusate pH toward the alkaline range above 8.0. The periods of infusion and pump flow rates are shown in the table:

| Patient | Weeks of Insulin Infusion | Flow Rate (ml/day) | |
|---|---|---|---|
| | | Initial | Final |
| SJ | 24 | 3.14 | 2.85 |
| SM | 34 | 3.00 | 3.00 |
| NP | 35 | 1.99 | 1.93 |
| JP | 35 | 2.28 | 2.00 |
| NB | 55 | 2.88 | 2.86 |
| DN | 67 | 3.42 | 3.21 |
| DM · | 67 | 3.21 | 3.05 |
| CS | 69 | 3.50 | 3.50 |
| CJ | 74 | 1.53 | 1.42 |
| Mean | 51 | 2.77 | 2.64 |
| Per cent change | | | −5 per cent |

Total patient/weeks of infusion: 460

## Example 2

Effect of incubation of the insulin/glycerol/bicarbonate mixture at various temperatures on insulin oligomer formation. To assess the effect of glycerol or other additives on the formation of higher apparent molecular weight species of insulin (insulin oligomers), the insulin/glycerol/bicarbonate mixture (40 units of insulin per ml) or insulin in regular insulin diluting fluid was incubated at 4°, 24° and 37°C in sterile glass vials for 0—4 weeks. In similar experiments, the insulin/glycerol/bicarbonate mixture was incubated in sealed infusion pumps under the same conditions. Following each incubation a sample from each vial was prepared for electrophoresis under non-reducing conditions by the addition of (final concentration): 1.0 per cent (w/v) sodium dodecyl sulfate (SDS), 3.3 per cent (w/v) sucrose; and .001 per cent (w/v) pyromin y. The samples were incubated at 37°C for 60 minutes, following which 25 μl of sample (containing 0.8 units of insulin) was applied to the lanes of polyacrylamide slab gels prepared according to Laemmli (Nature, 1970; 227: 680—685) except that gels were cast of 20 per cent (w/v) acrylamide/2.67 per cent methylene-bisacrylamide. Details of gel staining, destaining, densitometric scanning and protein molecular weight determination were as described by Blackshear et al (Biochem. J., 1982; 204: 817—824). Occasionally, insulin/glycerol/bicarbonate solutions removed from the pumps after a normal flow cycle in the patient studies were subjected to electrophoresis in the same way. Finally, the effect of disulfide-bond reduction on insulin-polymer molecular weight was studied by adding 40 mM (final concentration) dithiothreitol to the SDS electrophoresis buffer prior to the 37°C incubation.

Incubation of the insulin/glycerol/bicarbonate mixture in glass vials at various temperatures for 4 weeks resulted in the temperature-dependent formation of larger molecular weight species of insulin. By analysis using non-reducing electrophoresis, normal Neutral Regular porcine insulin (Lilly) contained approximately 2 per cent (by densitometry of stained protein) of a larger molecular weight species (about $M_r$ 7,300); this percentage increased only slightly after incubation at 37°C for one month.

In contrast, the percentage of larger molecular weight forms of insulin increased markedly after incubation of the insulin/glycerol/bicarbonate mixture for one month at 0°C, 24°C or 37°C. In addition, several still larger molecular weight forms appeared after incubation with glycerol at 24°C and 37°C. The formation of larger molecular weight forms of insulin in the presence of glycerol occurred to an identical extent during parallel incubations in glass vials and sealed implantable infusion pumps.

The proportion of insulin in the form of higher molecular weight species increased as a function of both time and temperature in the insulin/glycerol/bicarbonate mixture. At the end of four weeks of incubation in glass vials at 37°C, approximately 33 per cent of the insulin was in the form of higher molecular weight species.

Insulin removed from pumps after two weeks in patients displayed similar changes, i.e., there were larger proportions of higher molecular weight forms. When insulin previously incubated for four weeks at 37°C was subjected to electrophoresis under reducing conditions, the largest form visible had a $M_r$ slightly larger than native, unreduced insulin; in addition, large amounts of protein migrating near the dye front, presumably free A and B chains, could be detected.

## Example 3

Effect of buffering with sodium bicarbonate or sodium phosphate on the extent of insulin oligomer formation. After incubation in glass vials at 37°C for four weeks, sodium bicarbonate (12 mM) decreased the amount of insulin-polymer formation in 80 per cent glycerol when compared to insulin in glycerol without bicarbonate. Further addition of 54 mM sodium phosphate decreased the formation of polymers still further.

The biological activity of this insulin/glycerol/bicarbonate/phosphate mixture was tested in a dog previously made diabetic with alloxan according to the standard technique described by applicant and colleagues (Surg. Gynecol. Obstet., 1982; 155: 860—864). The insulin delivered intravenously in this animal from an implantable pump was either the insulin/glycerol/bicarbonate mixture described above, or an identical solution containing in addition (final concentration) 54 mM sodium phosphate, pH 7.0. To determine whether the insulin actually delivered from the pump's infusion cannula retains biological activity during a three week flow cycle, the effects of alternating cycles of phosphate buffered and un-buffered insulin on fasting plasma glucose levels were compared at insulin delivery rates of 22 units/day.

The phosphate-buffered solutions clearly prevented the gradual increase in fasting plasma glucose concentrations that occurred late in a three week flow cycle without phosphate. The addition of bicarbonate and phosphate buffers to the insulin/glycerol mixture appears to be largely effective in preventing the glycerol-induced formation of presumably inactive insulin oligomers.

Although the invention has been described with particular reference to the use of glycerol, other biocompatible C—4 to C—18 polyols, including sugars, behave similarly to glycerol as protectors of protein structure and function. Exemplary of such other polyols are C—4: erythritol; C—5: arabinose, xylose, ribose, adonital (ribitol) and arabitol; C—6: rhamose, inositol, fructose, galactose, glucose, mannose and sorbose, C—12: maltose and sucrose; and C—18: melezitose and raffinose. Where solid polyols are used they are dissolved in the standard aqueous insulin solution, or first prepared as an aqueous solution and admixed with the insulin, to provide a final concentration of polyol in the solution of 10 to 90% weight/volume, and preferably 40 to 80%. Similarly, although the invention is described with particular reference to solubilization of insulin, other infusible hormone are subject to the same precipitation problems, including growth hormone or glucagon.

The specific embodiments described are given by way of example and illustration of the invention.

## Claims

1. A protein hormone solution suitable for parenteral administration at a low flow rate to a chronically ill patient suffering from a hormone-deficiency disease from a drug delivery device that depends on the fluidity of the infusate for proper functioning, said solution comprising a polyol having 3 to 18 carbon atoms in an amount between 10 and 90 per cent by volume, effective to prevent precipitation of the protein hormone, and a buffer system at a concentration between 1 mMolar and 1000 mMolar sufficient to maintain a pH within one unit of the optimum pH of said protein hormone whereby the biological activity of the hormone-polyol solution is maintained during extended periods of storage within the drug device.

2. A solution according to claim 1 comprising from 40 to 90 per cent by volume of said polyol.

3. A solution according to claim 1 or claim 2 wherein the polyol is selected from glycerol, erythritol, arabinose, xylose, ribose, adonital (ribitol), arabitol, rhamose, inositol, fructose, galactose, glucose, mannose, sorbose, maltose, sucrose, melezitose and raffinose.

4. A solution according to any one of claims 1 or 3 wherein said polyol is glycerol.

5. A solution according to any of claims 1 to 4 wherein said buffer system is a phosphate buffer.

6. A solution according to any of claims 1 to 5 wherein said protein hormone is insulin.

7. An insulin solution suitable for parenteral administration at a low flow rate to a chronically ill patient suffering from diabetes from a drug delivery device that depends on the fluidity of the infusate for proper functioning said solution comprising a polyol in an effective amount between 40 and 90 per cent by volume, effective to prevent precipitation of the insulin, and a sodium phosphate buffer system in concentration between 1 mMolar and 1000 mMolar sufficient to maintain a pH within one unit of the optimum pH of said insulin whereby the biological activity of the insulin-polyol solution is maintained during extended periods of storage within the drug device.

8. A solution according to claim 7 wherein said polyol is glycerol and said buffer system is sodium phosphate in combination with sodium bicarbonate.

9. A solution according to any of the preceding claims contained in the storage chamber of an implanted drug delivery device.

## Patentansprüche

1. Proteinhormonlösung zur parenteralen Verabreichung bei niedriger Flußrate über eine Medikamentenabgabevorrichtung, deren richtige Funktion von der Fluidität des Infusates abhängig ist, an einen chronisch kranken Patienten, der an einer Hormonmangelkrankheit leidet, wobei die Lösung ein Polyol mit 3—18 Kohlenstoffatomen in einer Menge von 10—90 Vol.% umfaßt, die ein Ausfällen des Proteinhormons verhindert, und ein Puffersystem in einer Konzentration von 1 mMolar bis 1000 mMolar umfaßt, die ausreicht, um den pH innerhalb etwa einer Einheit um das pH Optimum des Proteinhormons zu halten, wobei die biologische Aktivität der Hormon-Polyollösung während längerer Aufbewahrungszeiträume in der Medikamentenvorrichtung beibehalten wird.

2. Lösung nach Anspruch 1, welche 40—90 Vol.% des Polyols umfaßt.

3. Lösung nach Anspruch 1 oder Anspruch 2, worin das Polyol ausgewählt ist unter Glycerin, Erythritol, Arabinose, Xylose, Ribose, Adonital (Ribitol), Arabitol, Rhamnose, Inositol, Fructose, Galactose, Glucose, Mannose, Sorbose, Maltose, Sucrose, Melezitose und Raffinose.

4. Lösung nach einem der Ansprüche 1 oder 3, worin das Polyol Glycerin ist.

5. Lösung nach einem der Ansprüche 1 bis 4, worin das Puffersystem ein Phosphatpuffersystem ist.

6. Lösung nach einem der Ansprüche 1 bis 5, worin das Proteinhormon Insulin ist.

7. Insulinlösung zur parenteralen Verabreichung bei niedriger Flußrate über eine Medikamentenabgabevorrichtung, deren richtige Funktion von der Fluidität des Infusates abhängig ist, an einen chronisch erkrankten Patienten, der an Diabetes leidet, wobei die Lösung ein Polyol in einer wirksamen Menge von 40—90 Vol.% umfaßt, die eine Ausfällung des Insulins verhindert, und ein Natriumphosphatpuffersystem in einer Konzentration von 1 mMolar bis 1000 mMolar umfaßt, die ausreicht, um den pH innerhalb einer Einheit um das pH Optimum des Insulins zu halten, wobei die biologische Aktivität der Insulin-Polyollösung während längerer Aufbewahrungszeiträume in der Medikamentenvorrichtung beibehalten wird.

8. Lösung nach Anspruch 7, worin das Polyol Glycerin und das Puffersystem Natriumphosphat in Kombination mit Natribicarbonat ist.

9. Lösung nach einem der vorhergehenden Ansprüche, welche in einer Speicherkammer einer implantierten Medikamentenabgabevorrichtung enthalten ist.

## Revendications

1. Solution d'hormone protéique convenable pour l'administration parentérale, à faible débit, à un patient souffrant d'une maladie chronique consistant en un déficit hormonal, par un dispositif d'administration de médicament dont le fonctionnement convenable dépend de la fluidité du liquide de perfusion, ladite solution comprenant un polyol ayant 3 à 18 atomes de carbone en une quantité de 10 à 90% en volume, efficace pour empêcher la précipitation de l'hormone protéique, et un système-tampon à une concentration de 1 mmolaire à 1000 mmolaires, suffisante pour maintenir le pH approximativement au pH optimal, plus ou moins une unité, de ladite hormone protéique, ce qui permet de maintenir l'activité biologique de la solution hormone-polyol au cours de longues périodes d'entreposage à l'intérieur du dispositif d'administration de médicament.

2. Solution suivant la revendication 1, comprenant 40 à 90% en volume du polyol.

3. Solution suivant la revendication 1 ou la revendication 2, dans laquelle le polyol est choisi entre le glycérol, l'érythritol, l'arabinose, le xylose, le ribose, l'adonitol (ribitol), l'arabitol, le rhamnose, l'inositol, le fructose, le galactose, le glucose, le mannose, le sorbose, le maltose, le saccharose, le mélézitose et le raffinose.

4. Solution suivant l'une quelconque des revendications 1 à 3, dans laquelle le polyol est le glycérol.

5. Solution suivant l'une quelconque des revendications 1 à 4, dans laquelle le système-tampon est un tampon au phosphate.

6. Solution suivant l'une quelconque des revendications 1 à 5, dans laquelle l'hormone protéique est l'insuline.

7. Solution d'insuline convenable pour l'administration parentérale, à faible débit à un patient souffrant d'une maladie chronique consistant en un diabète, par un dispositif d'administration de médicament dont le fonctionnement convenable dépend de la fluidité du liquide de perfusion, ladite solution comprenant un polyol en une quantité de 40 à 90% en volume, efficace pour éviter la précipitation de l'insuline, et un système-tampon au phosphate de sodium en une concentration de 1 mmolaire à 1000 mmolaires, suffisante pour maintenir le pH approximativement au pH optimal, plus ou moins une unité, de ladite insuline, ce qui permet de maintenir l'activité biologique de la solution insuline-polyol au cours de longues périodes d'entreposage à l'intérieur du dispositif d'administration de médicament.

8. Solution suivant la revendication 7, dans laquelle le polyol est le glycérol et le système-tampon est un tampon au phosphate de sodium en association avec du bicarbonate de sodium.

9. Solution suivant l'une quelconque des revendications précédentes, placée dans le compartiment d'entreposage d'un dispositif implanté d'administration de médicament.